# EUROPÄISCHE PATENTSCHRIFT

(11) **EP 1 849 434 B1**
(45) Veröffentlichungstag und Bekanntmachung des Hinweises auf die Patenterteilung: **03.07.2013**
(21) Anmeldenummer: 07008070.0
(22) Anmeldetag: 20.04.2007
(51) Int. Cl.: A61F 2/00, A61F 2/06, A61K 31/727, A61K 38/04, A61K 38/58, A61L 27/00, A61L 29/00, A61L 33/00, A61M 25/00, A61M 25/10, A61K 9/00, A61F 2/82

(54) **Beschichtung für Gefäßprothesen**
Coating for stents
Revêtement pour prothèses vasculaires

(30) Priorität: 21.04.2006 DE 102006018630
(43) Veröffentlichungstag der Anmeldung: 31.10.2007
(73) Patentinhaber: Stemberger, Axel, 85579 Neubiberg (DE); Schmidmaier, Gerhard,, 10717 Berlin (DE); Raschke, Michael, 48145 Münster (DE)
(72) Erfinder: Stemberger, Axel, 85579 Neubiberg (DE); Schmidmaier, Gerhard,, 10717 Berlin (DE); Raschke, Michael, 48145 Münster (DE)
(74) Vertreter: Czybulka, Uwe

(56) Entgegenhaltungen:
- EP-A- 0 756 853
- DE-A1- 4 435 652
- DE-A1- 19 514 104
- DE-A1- 19 718 339

## Beschreibung

Die Erfindung bezieht sich auf Verwendungen von Beschichtungen für Gefäßprothesen und insbesondere auf eine Beschichtung für Prothesen aus Polytetrafluorethylen, so genannte PTFE-Prothesen.

Solche Prothesen ersetzen ein natürliches Gefäß des menschlichen Körpers und werden zum Beispiel als Bypass-Prothesen in der peripheren Gefäßchirurgie etwa im Koronarbereich eingesetzt.

Bei der Implantation derartiger Gefäßprothesen muss Sorge dafür getragen werden, dass im Rahmen der operativen Implantation thrombotische Verschlüsse der Gefäßprothese verhindert werden, die in den ersten ca. zwei bis vier Wochen nach der Implantation mit einem relativ hohen Prozentsatz auftreten.

Es ist zum Beispiel für Stützprothesen, sogenannte Stents, die im Wesentlichen aus einem aufweitbaren Maschendrahtgerüst bestehen, bekannt, diese Stents mit einer dünnen Beschichtung zu versehen, die auf dem Stent selbst haftet und eine lackartige Haftschicht mit geringen Schichtdicken von kleiner als 100 µm bildet, vgl. die EP 0652017 B2. In diese Beschichtung können zusätzliche, die Blutgerinnung hemmende Arzneistoffe eingearbeitet sein, vorzugsweise Hirudin und ggf. in Kombination hiermit Iloprost, wobei diese Arzneistoffe plasmatische und zelluläre Blutgerinnung hemmende Stoffe sind. Mit solchen Beschichtungen können die akuten Gerinnungsproblematiken nach der Implantation relativ gut beherrscht werden.

Von diesen akuten Gerinnungsproblematiken sind jedoch die chronischen Veränderungen zu unterscheiden, die etwa in PTFE-Bypässen immer auftreten. Diese chronischen Veränderungen betreffen im Wesentlichen die Ausbildung einer Pseudointima, die innerhalb der Gefäßprothese, zum Beispiel eines Bypasses auftritt, und die Intimahyperplasie, die außerhalb der Gefäßprothese innerhalb der arteriellen Empfängersegmente in der Nähe der Anastomose auftritt. Beide Phänomene sind als unabhängig voneinander entstehende Entitäten zu betrachten, die primär nicht nur mit Gerinnungsvorgängen in Verbindung gebracht werden können.

Eine Pseudointimaentwicklung beginnt in der Regel innerhalb von wenigen Tagen nach der Implantation der Gefäßprothese; sie besteht aus luminalen thrombotischen Ablagerungen aus aktivierten Thrombozyten, Erytrozyten, Fibrin und degenerierten Zellen. Diese Ablagerungen werden von Makrophagen aus dem Blutstrom phagozytiert. Das Endresultat ist eine weiche Matrix, die sich über das gesamte Innenlumen der Bypassoberfläche erstreckt und später von Endothelzellen besiedelt wird. Die Pseudointima wächst im Verlauf von mehreren Wochen beziehungsweise Monaten weiter und kann zu einem Verschluss der Gefäßprothese, zum Beispiel eines Bypasses, führen, obwohl Arzneimittel zur Verhinderung einer Koagulation verabreicht wurden.

Eine Intimahyperplasie, die sich außerhalb der Gefäßprothese, zum Beispiel eines Bypasses bildet, ist eine Anpassungsreaktion der Gefäßwand auf zu niedrige Scherstresse bzw. Scherraten, die unabhängig von thrombotischen Prozessen ist. Endothelzellen sind offensichtlich in der Lage, über verschiedene Rezeptorsysteme den Scherstress zu "messen". Ein bestimmtes Scherstress-Niveau ist für die Aufrechterhaltung der normalen Gefäßwandhomöotase erforderlich. Bei niedrigem Wandscherstress werden subendotheliale Fibrozyten aktiviert, die zu einer subendothelialen Hyperplasie und damit zu einer Verdickung der Gefäßwand führen. Folge ist, dass die Endothelzellen wieder einem höheren Wandscherstress ausgesetzt sind, was zu einer Aktivierung von weiteren Fibrozyten führt. Diese Ausbildung einer Intimahyperplasie ist unabhängig von thrombotischen Wirkungen.

Die deutsche Offenlegungsschrift DE 195 14 104 offenbart Beschichtungen für Stützkonstruktionen für Gefässe enthaltend neben dem Antithrombin (CRC220) auch Hirudin sowie Iloprost.

Die deutsche Offenlegungsschrift DE 44 35 652 offenbart ebenfalls Beschichtungen für in den Blutstrom oder in das Gewebe des menschlichen Körpers einbringbares Biomaterial wie z.B. Gefässprothesen oder Stützkörper für Gefäße, Stents, die eine Schichtdicke kleiner als 100 Mikrometer aufweisen sowie eine Hirudin beinhalten und/oder Iloprost.

Der Erfindung liegt die Aufgabe zugrunde, eine Beschichtung für eine Gefäßprothese der in Rede stehenden Art anzugeben, mit der zuverlässig chronische Veränderungen in dieser Gefäßprothese verhindert werden beziehungsweise deutlich reduziert werden, insbesondere somit die Ausbildung einer Pseudointima und einer Intimahyperplasie.

Es hat sich nun überraschend herausgestellt, dass eine Beschichtung der Gefäßprothese bei Verwendung einer Kombination eines eingearbeiteten Antithrombotikums und eines Plättchenaggregationshemmers die Bildung einer Pseudointima beziehungsweise einer Intimahyperplasie verhindert beziehungsweise deutlich reduziert. Bei einer Verwendung dieser Wirkstoffe in einer Beschichtung für Gefäßprothesen gemäß der Erfindung werden diese chronischen Prozesse verhindert, und zwar auch in Gefäßprothesen wie z.B. Bypässen, die nur ein kleines Lumen aufweisen.

Die Kombination aus einem Antithrombotikum und einem Plättchenaggregationshemmer in einer Beschichtung für Gefäßprothesen sind aus der oben erwähnten EP 0652017 A1 zwar bekannt; diese Wirkstoffe wurden aber nur entsprechend ihrer bekannten Wirkung zur Hemmung plasmatischer und zellulärer Blutgerinnung eingesetzt. Es war daher für einen Fachmann überraschend, dass mit einer solchen Beschichtung auch chronische Veränderungen in einer Gefäßprothese, eben die Entwicklung einer Pseudointima und einer Intimahyperplasie, die auf anderen Ursachen beruhen, verhindert bzw. merklich gehemmt werden können.

Diese überraschende Wirkung konnte bereits klinisch in Tierversuchen nachgewiesen werden.

Als Antithrombotikum werden vorzugsweise Hirüdin bzw. PEG-Hirudin oder ein anderer direkt wirkender Inhibitor von Thrombin bzw. ein direkt wirkender Inhibitor des Faktors Xa und als Plättchenaggregationshemmer vorzugsweise Iloprost verwendet.

Das Hirudin wird mit Iloprost vermischt und in dieser Kombination in einen Träger vorzugsweise aus einem Polylactid-Polymer eingearbeitet.

In Versuchen wurden zum Beispiel 8 Zentimeter lange Segmente einer PTFE-Gefäßprothese mit 4 Millimeter Durchmesser unter sterilen Bedingungen mit einem Polylactid-Polymer (R203 der Firma Boehringer Ingelheim, Deutschland), gelöst in einem flüchtigenorganischen Lösungsmittel, z: B. Chloroform, beschichtet. Es wurde eine 8 %-ige Lösung eines Polylactid-Polymers in Chloroform verwendet, die 5 % PEG-Hirudin und 1 % Iloprost (zum Beispiel Ilomedin ® der Schering AG, Berlin, Deutschland) enthielt. Die Prozentzahlen sind jeweils Gewichtsprozente. Die Gefäßprothesen wurden unter aseptischen Bedingungen zweimal in die Lösung getaucht, um eine homogene Beschichtung zu erhalten, anschließend getrocknet und steril verpackt. Nach einer anfänglich relativ hohen Freigabe des Antithrombotikums Hirudin während der ersten 48 Stunden wurde eine langsamere und kontinuierliche Freigabe über eine Zeitspanne von mehr als drei Monaten beobachtet. Nach 90 Tagen waren etwa 60 % des PEG-Hirudin und 10 % des Iloprost freigesetzt.

Nach dieser Zeit konnte bei den beschichteten Gefäßprothesen lediglich eine dünne und transparente Schicht einer Pseudointima beobachtet werden. Bei allen Gefäßprothesen wurde eine Abnahme der Hyperplasie mit größeren Entfernungen von der Anastomose festgestellt.

Es wurden keine negativen lokalen oder systemischen Wirkungen der Beschichtung der Gefäßprothese beobachtet. Die Beschichtung beeinflusste nicht das Einheilen der Gefäßprothese in das subkutane Gewebe; ebenso wurden keine Reaktionen um die Gefäßprothese beobachtet. Die lokal freigegebenen Arzneistoffe führen zu keiner Veränderung der globalen Hämostase, also Funktion der Thrombozyten sowie der plasmatischen Gerinnung. Dieses unterstützt die Hypothese, dass die Wirkung der gelösten Arzneistoffe im Wesentlichen auf die Oberfläche der Gefäßprothese beschränkt bleibt.

## Patentansprüche

1. Eine Kombination eines Antithrombotikums und eines Plättchenaggregationshemmers in einer Beschichtung für Gefäßprothesen, insbesondere Prothesen aus Polytetrafluorethylen (PTFE-Prothesen), durch die ein natürliches Gefäß des menschlichen Körpers ersetzt wird, Verwendung zur Behandlung einer Pseudointima im Inneren der Gefäßprothese und einer Intimahyperplasie außerhalb der Gefäßprothese.

2. Eine Kombination zur Behandlung nach Anspruch 1, **dadurch gekennzeichnet, dass** das Antithrombotikum ein direkt wirkender Thrombininhibitor und/oder ein direkt wirkender Xa-Inhibitor ist.

3. Eine Kombination zur Behandlung nach Anspruch 1 oder 2, **dadurch gekennzeichnet, dass** das Antiththrombotikum Hirudin oder PEG-Hirudin ist.

4. Eine Kombination zur Behandlung nach einem der vorhergehenden Ansprüche, **dadurch gekennzeichnet, dass** der Plättchenaggregationshemmer Iloprost ist.

5. Eine Kombination zur Behandlung nach einem der vorhergehenden Ansprüche, **dadurch gekennzeichnet, dass** das Antithrombotikum und der Plättchenaggregationshemmer in einen Träger, vorzugsweise ein Polylactid, eingearbeitet sind.

6. Eine Kombination zur Behandlung nach einem der vorhergehenden Ansprüche, **dadurch gekennzeichnet, dass** die Beschichtung mit dem eingearbeiteten Antithrombotikum und dem eingearbeiteten Plättchenaggregationshemmer insbesondere für Gefäßprothesen mit kleinem Lumen verwendet wird.

7. Eine Kombination zur Behandlung nach Anspruch 6, **dadurch gekennzeichnet, dass** das Lumen einen Durchmesser bis 10 Millimeter, vorzugsweise bis 5 Millimeter aufweist.

## Claims

1. A combination of an antithrombotic and a platelet aggregation inhibitor in a coating for vascular prostheses, in particular prostheses made of polytetrafluoroethylene (PTFE prostheses) which replace a natural vessel of the human body, to be used for treating a pseudointima in the interior of the vascular prosthesis and an intima hyperplasia outside the vascular prosthesis.

2. The combination for treatment according to claim 1, **characterized in that** the antithrombotic is a directly acting thrombin inhibitor and/or a directly acting Xa inhibitor.

3. The combination for treatment according to claim 1 or 2, **characterized in that** the antithrombotic is hirudin or PEG hirudin.

4. The combination for treatment acccording to any of the preceding claims, **characterized in that** the platelet aggregation inhibitor is iloprost.

5. The combination for treatment according to any of the preceding claims, **characterized in that** the antithrombotic and the platelet aggregation inhibitor are incorporated into a carrier, preferably a polylactide.

6. The combination for treatment according to any of the preceding claims, **characterized in that** the coating with the incorporated antithrombotic and the incorporated platelet aggregation inhibitor is in particular used for vascular prostheses with a small lumen.

7. The combination for treatment according to claim 6, **characterized in that** the lumen has a diameter of up to 10 mm, preferably up to 5 mm.

## Revendications

1. Une combinaison d'un antithrombotique et d'un antiagrégant plaquettaire dans un revêtement pour des prothèses vasculaires, en particulier des prothèses en polytétrafluoroéthylène (prothèses PTFE) par lesquelles un vaisseau sanguin naturel du corps humain est remplacé, destinée à être utilisée pour le traitement d'une pseudo-intima à l'intérieur d'une prothèse vasculaire et d'une hyperplasie d'intima à l'extérieur de la prothèse vasculaire.

2. Une combinaison pour traitement selon la revendication 1, **caractérisée en ce que** l'antithrombotique est un inhibiteur direct de la thrombine et/ou un inhibiteur direct du facteur Xa.

3. Une combinaison pour traitement selon la revendication 1 ou 2, **caractérisée en ce que** l'antithrombotique est de l'hirudine ou de la PEG-hirudine.

4. Une combinaison pour traitement selon l'une des revendications précédentes, **caractérisée en ce que** l'antiagrégant plaquettaire est de l'iloprost.

5. Une combinaison pour traitement selon l'une des revendications précédentes, **caractérisée en ce que** l'antithrombotique et l'antiagrégant plaquettaire sont incorporés dans un support, de préférence dans un polylactide.

6. Une combinaison pour traitement selon l'une des revendications précédentes, **caractérisée en ce que** le revêtement avec l'antithrombotique incorporé et l'antiagrégant plaquettaire incorporé est utilisé en particulier pour des prothèses vasculaires avec petit lumen.

7. Une combinaison pour traitement selon la revendication 6, **caractérisée en ce que** le lumen présente un diamètre allant jusqu'à 10 millimètres, de préférence jusqu'à 5 millimètres.
